# EUROPÄISCHE PATENTSCHRIFT

(11) **EP 1 248 780 B1**
(45) Veröffentlichungstag und Bekanntmachung des Hinweises auf die Patenterteilung: **30.03.2005**
(21) Anmeldenummer: 01909498.6
(22) Anmeldetag: 19.01.2001
(51) Int. Cl.: C07D 333/36, C07D 409/04, C07D 409/14

(54) **DI(HET)ARYLAMINOTHIOPHEN-DERIVATE**
DI(HET)ARYLAMINOTHIOPHENE DERIVATIVES
DERIVES DI(HET)ARYLAMINOTHIOPHENES

(30) Priorität: 20.01.2000 DE 10002424
(43) Veröffentlichungstag der Anmeldung: 16.10.2002
(73) Patentinhaber: SIEMENS AKTIENGESELLSCHAFT, 80333 München (DE)
(72) Erfinder: ROGLER, Wolfgang, 91096 Möhrendorf (DE); KANITZ, Andreas, 91333 Höchstadt (DE); HARTMANN, Horst, 06217 Merseburg (DE); SCHUMANN, Jörg, 91056 Erlangen (DE)
(74) Vertreter: Epping Hermann & Fischer
(86) Internationale Anmeldenummer: PCT/DE2001/000226
(87) Internationale Veröffentlichungsnummer: WO 2001/053286

(56) Entgegenhaltungen:
- PATENT ABSTRACTS OF JAPAN vol. 012, no. 460 (P-795), 5. Dezember 1988 (1988-12-05) & JP 63 183451 A (FUJI ELECTRIC CO LTD), 28. Juli 1988 (1988-07-28)
- PATENT ABSTRACTS OF JAPAN vol. 017, no. 557 (P-1626), 7. Oktober 1993 (1993-10-07) & JP 05 158260 A (FUJI ELECTRIC CO LTD), 25. Juni 1993 (1993-06-25)
- PATENT ABSTRACTS OF JAPAN vol. 1998, no. 13, 30. November 1998 (1998-11-30) & JP 10 219242 A (SHIROTA YASUHIKO;TDK CORP), 18. August 1998 (1998-08-18)

## Beschreibung

Die Erfindung betrifft neue Di(het)arylaminothiophen-Derivate, d.h. Diarylaminothiophen- bzw. Dihetarylaminothiophen-Derivate ("hetaryl" = "heteroaryl"), sowie deren Herstellung und Verwendung.

Für organische lichtemittierende Dioden (organische LEDs = OLEDs) und organische photovoltaische Bauelemente werden organische Materialien benötigt, die zur Elektrolumineszenz befähigt sind. Dies können entweder Verbindungen mit geringer Molekülgröße sein (siehe beispielsweise US-PS 4 539 507), die verdampfbar sind, oder polymere Materialien (siehe beispielsweise US-PS 5 247 190), die durch Spin-coating verarbeitet werden können. JP 10-219242 (18.08.1998) beschreibt Oligothiophene zum Aufbau organischer lichtemittierender Bauelemente.

Die Synthese von Verbindungen der genannten Art erfordert Aromatenkupplungsreaktionen. Derartige Reaktionen, bei denen halogenhaltige Verbindungen eingesetzt werden, verlaufen zum Teil unter Metallkatalyse, beispielsweise nach einer Heck-Reaktion oder nach einer Suzuki-Reaktion (siehe dazu: "Chem. Commun.", 1999, Seiten 1837 bis 1838). Hierbei ist es aber kaum möglich, das Metall, wie Palladium (siehe dazu: "Römpp Chemie-Lexikon", 9. Auflage, Seite 1750), vollständig zu entfernen. Metalle - und auch Spuren von nicht vollständig umgesetzten halogenhaltigen Zwischenprodukten - wirken aber als sogenannte Quentcher, d.h. sie löschen in ihrer Umgebung die Elektrolumineszenz, und sie setzen deshalb die Effizienz der hergestellten Materialien stark herab.

Bei einer Aromatenkupplung entsprechend einer Ullmann-Reaktion (siehe dazu: "Römpp Chemie-Lexikon", 9. Auflage, Seite 4796) ist die Bildung von Nebenprodukten und von Crackprodukten, die infolge hoher Prozesstemperaturen entstehen, ein großes Problem. Hierbei ist nämlich die Reinigung des Reaktionsproduktes in vertretbarem Ausmaß kaum oder überhaupt nicht möglich.

Gegenstand der Erfindung sind deshalb neue 3,4-substituierte 2-(N,N-Di(het)arylamino)-thiophen-Derivate der allgemeinen Struktur 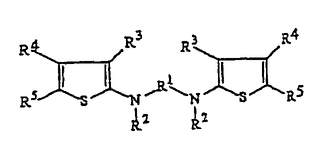 und 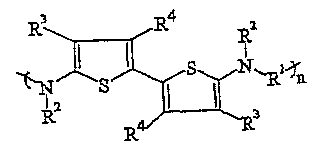 wobei Folgendes gilt:
R², R³, R⁴ und R⁵ sind - unabhängig voneinander - jeweils ein monofunktionelles (Het)arylsystem in Form eines konjugierten carbocyclischen oder heterocyclischen Ringsystems, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylamiogruppen (Alkyl = C₁ bis C₆) substituiert sein können und R¹ ist ein entsprechendes bifunktionelles (Het)arylensystem, und
wobei n = 2 bis 100 und eine ganze Zahl ist.

Außerdem sind Gegenstand der Erfindung 2-(N,N-Di(het)arylamino)thiophen Derivate folgender Strukturen.

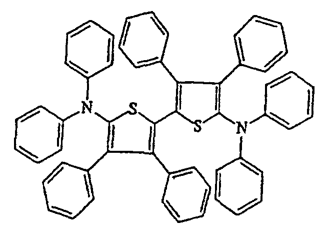

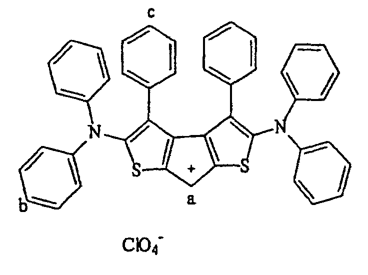

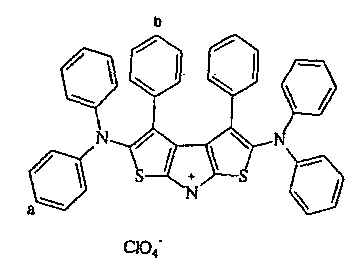

Schließlich ist Gegenstrand der Erfindung ein Verfahren zur Herstellung von Thiophen-Derivaten, **gekennzeichnet** durch folgende Schritte:
(a) Umsetzung eines sekundären Amins mit einem Carbonsäurehalogenid zu einem Carbonsäureamid;
(b) Überführung des Carbonsäureamids in ein Thiocarbonsäureamid;
(c) Umsetzung des Thiocarbonsäureamids mit einer α-Halogenacyl-Verbindung zu einem 2-(N,N-Di(het)arylamino)-thiophen-Derivat.

Die Herstellung der neuen Di(het)arylaminothiophen-Derivate erfolgt mittels einer Hetarylringschlussreaktion unter milden Bedingungen und ohne Metallkatalysatoren. Diese Verbindungen können somit in hoher Reinheit hergestellt werden, d.h. die Effizienz dieser Materialien, insbesondere die Elektrolumineszenz, wird nicht durch Verunreinigungen beeinträchtigt, die bei nach bekannten Verfahren hergestellten Verbindungen beispielsweise wegen des eingesetzten Katalysators vorhanden sind. Ein weiterer Vorteil dieser Materialien besteht in verbesserten und für den jeweiligen Zweck anpassbaren Redox-Eigenschaften, bedingt durch die Vielfalt der Strukturmöglichkeiten, die sich aus dem nachfolgend näher geschilderten Aufbauprinzip ergibt. Dieses Aufbauprinzip ermöglicht es ferner, dass die neuen Materialien problemlos auch in Form von Oligomer- und Polymer-Strukturen erhalten werden können, d.h. dass Oligo- und Poly-aminothiophen-Derivate herstellbar sind.

Die Synthese der neuen Verbindungen erfordert zum Teil Vorprodukte, die bislang nicht bekannt sind. Diese Vorprodukte sind aber - aus kommerziell erhältlichen Edukten - in nahezu quantitativer Ausbeute erhältlich.

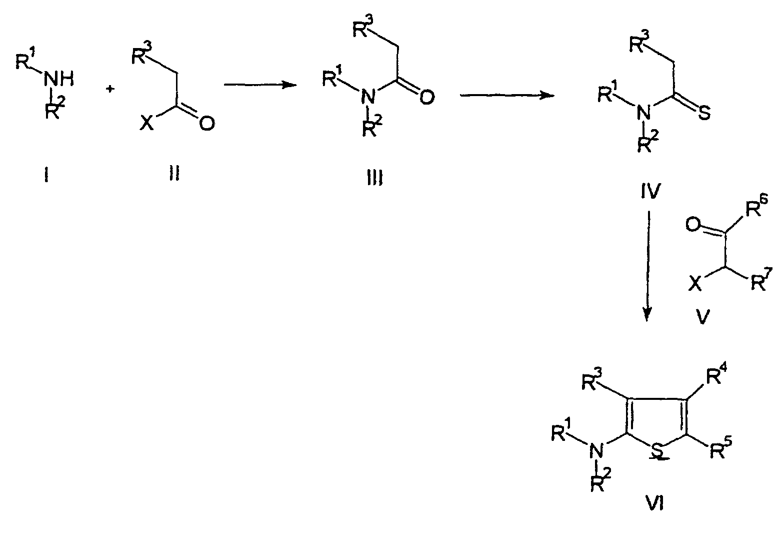

Hierbei gilt Folgendes:
R¹, R², R³, R⁴ und R⁵ haben die vorstehend angegebene Bedeutung;
R⁶ und R⁷ sind - unabhängig voneinander - jeweils ein monofunktionelles (Het)arylsystem, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylaminogruppen (Alkyl = C₁ bis C₆) substituiert sein können;
R⁶ kann ferner die Gruppierung R⁹ sein, wobei die Verbindung V an der freien Bindung eine Gruppe -CO-CH₂X trägt mit X = Halogen, vorzugsweise Cl, Br oder J,
oder R⁶ kann eine Gruppe -C(CO-CH₂X)₃ sein.

Die erste Stufe der Synthese der Thiophen-Derivate besteht in der Umsetzung eines sekundären Amins I mit einem Carbonsäurehalogenid II zu einem Carbonsäureamid (Säureamid) III. Die Umsetzung erfolgt in einem geeigneten Lösemittel, vorzugsweise Dioxan, bei erhöhter Temperatur, vorzugsweise im Bereich des Siedepunktes des Lösemittels, unter Inertgas. Die Reaktion ist dann beendet, wenn das als Nebenprodukt entstehende Hydrohalogenid (Halogenwasserstoff) durch den Inertgasstrom entfernt ist oder wenn kein Amin mehr nachweisbar ist, beispielsweise durch eine dünnschichtchromatographische Kontrolle.

Das Säureamid III wird dann in einer zweiten Stufe in ein Thiocarbonsäureamid (Thioamid) IV überführt. Dies erfolgt vorteilhaft mittels des so genannten Lawesson-Reagenz, einem in homogener Phase wirkenden Reagenz zur Einführung von Schwefel in Carbonylverbindungen (siehe dazu: "Römpp Chemie-Lexikon", 9. Auflage, Seite 2464), in einem geeigneten Lösemittel, vorzugsweise Diglykoldiethylether, bei erhöhter Temperatur, vorzugsweise bei ca. 100°C; Dauer: mehrere Stunden, im allgemeinen ca. 6 h. Die Einführung des Schwefels kann aber auch in heterogener Phase erfolgen, beispielsweise mittels Phosphorpentasulfid.

In einer dritten Stufe wird das Thioamid IV - je nach Substitutionsmuster (siehe nachfolgend IVa bis IVc) - durch Umsetzung mit einer α-Halogen-acylverbindung V in einem geeigneten Lösemittel, vorzugsweise Methylenchlorid, Dichlorethan, Acetanhydrid, Dimethylformamid oder Tetrahydrofuran, in ein halbleitendes 3,4-substituiertes 2-(N,N-Di(het)arylamino)-thiophen-Derivat VI überführt, und zwar je nach Substitutionsmuster der Acylverbindung (siehe nachfolgend Va bis Vd). Dies erfolgt durch eine primäre S-Alkylierung sowie eine nachfolgende Cyclisierung (Ringschlussreaktion) und Aromatisierung. Die Cyclisierung kann durch Zugabe eines deprotonierend wirkenden Agens, vorzugsweise Triethylamin, beschleunigt werden.

Thiophen-Derivate VI, die in 5-Stellung unsubstituiert sind, d.h. R⁵ = H, können durch oxidative Kupplung in dimere bzw. polymere Derivate VI umgewandelt werden (siehe nachfolgend VIg,h,i,n,r,s,t,v und z), die ebenfalls organische Halbleitermaterialien darstellen. Die oxidative Kupplung erfolgt in einem geeigneten Lösemittel, vorzugsweise trockenem Tetrahydrofuran, durch an sich bekannte Oxidationsmittel, vorzugsweise durch Oxidation des jeweiligen - mittels Butyllithium hergestellten - lithiierten Thiophen-Derivates mit Kupfer-II-chlorid oder durch Elektrooxidation auf einem leitfähigen Substrat, beispielsweise auf einer mit ITO beschichteten Glasscheibe (ITO = Indium Tin Oxide).

Mittels halogenierten 1,2-Diketonen (siehe nachfolgend Vd/d) - als α-Halogen-acylverbindung - aus den Thiocarbonsäureamiden IV hergestellte Thiophen-Derivate VI, die in 5,5'-Stellung unsubstituiert und in 4,4'-Stellung verbunden sind (siehe nachfolgend VIw und VIx), können mit Orthoameisensäureester oder mit salpetriger Säure, die beispielsweise aus Natriumnitrit oder Isoamylnitrit generiert wird, in entsprechende kationische Lochtransportmaterialien überführt werden (siehe nachfolgend VI/1 und VI/2), die den bekannten Polythiophenen und Polyanilinen in deren Eigenschaft als organische Leiter analog sind.

Nachfolgend sind die Strukturformeln einer größeren Anzahl der neuen Thiophen-Derivate - zusammen mit den unmittelbaren Ausgangsprodukten - in tabellarischer Form wiedergegeben.

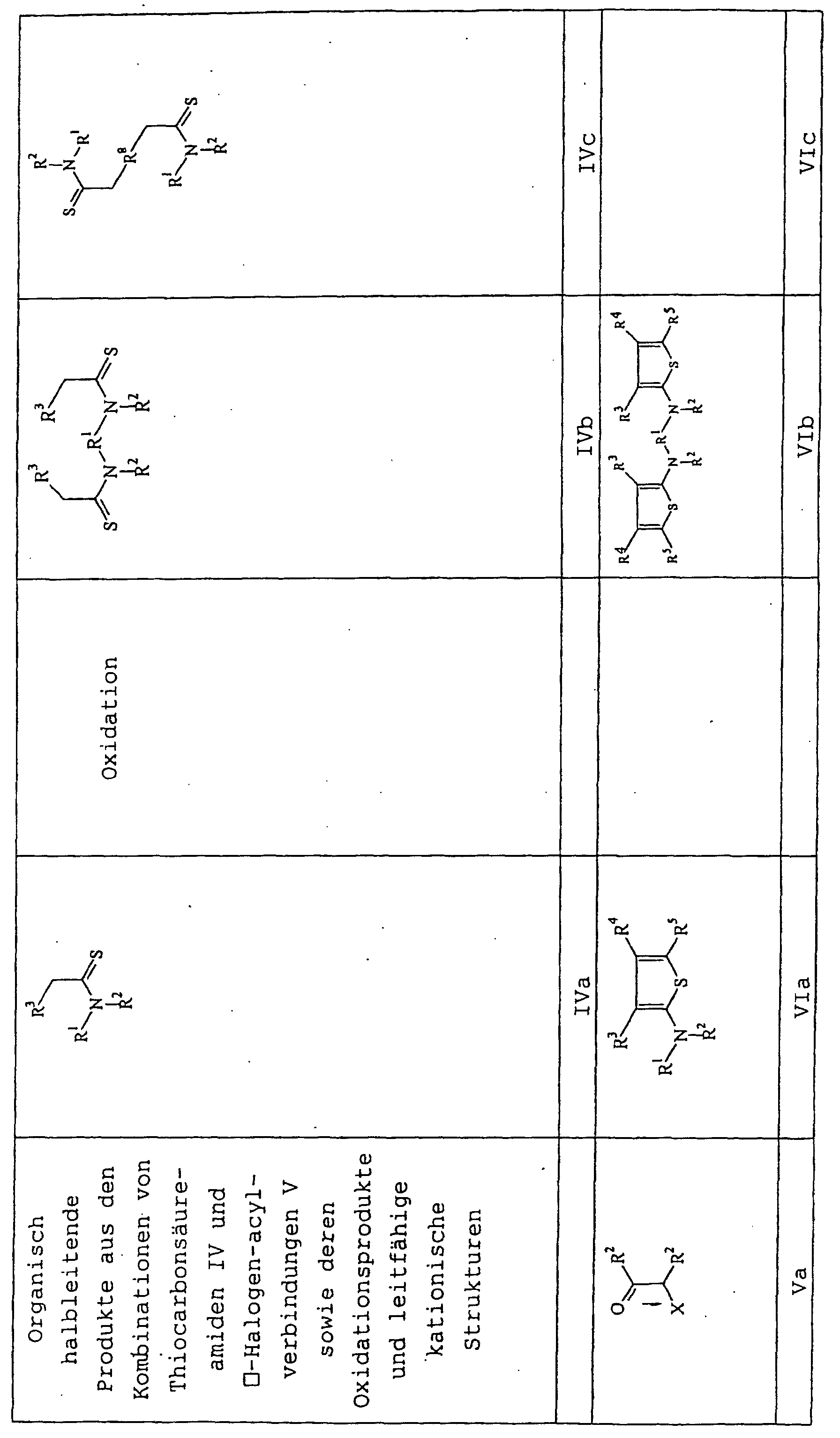

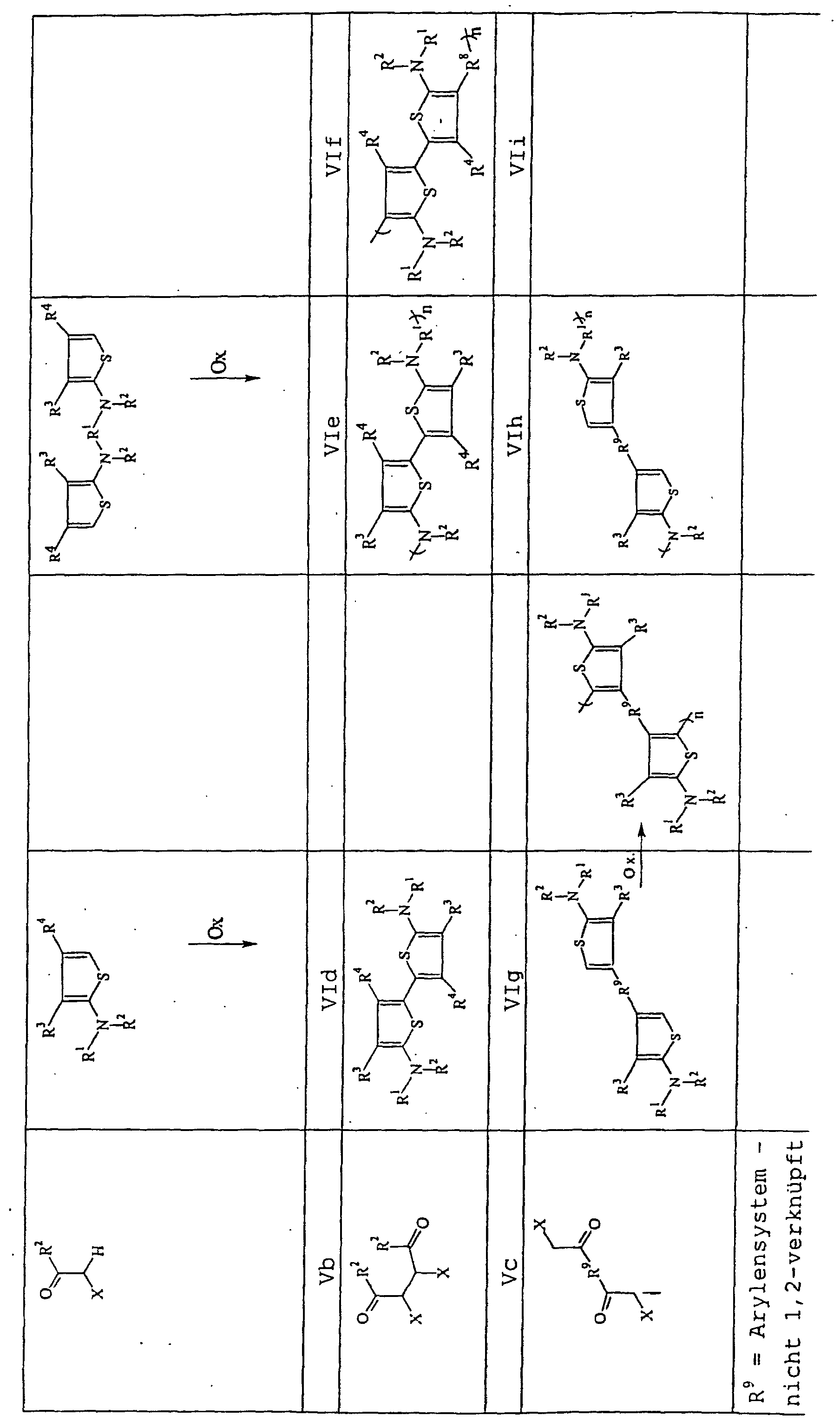

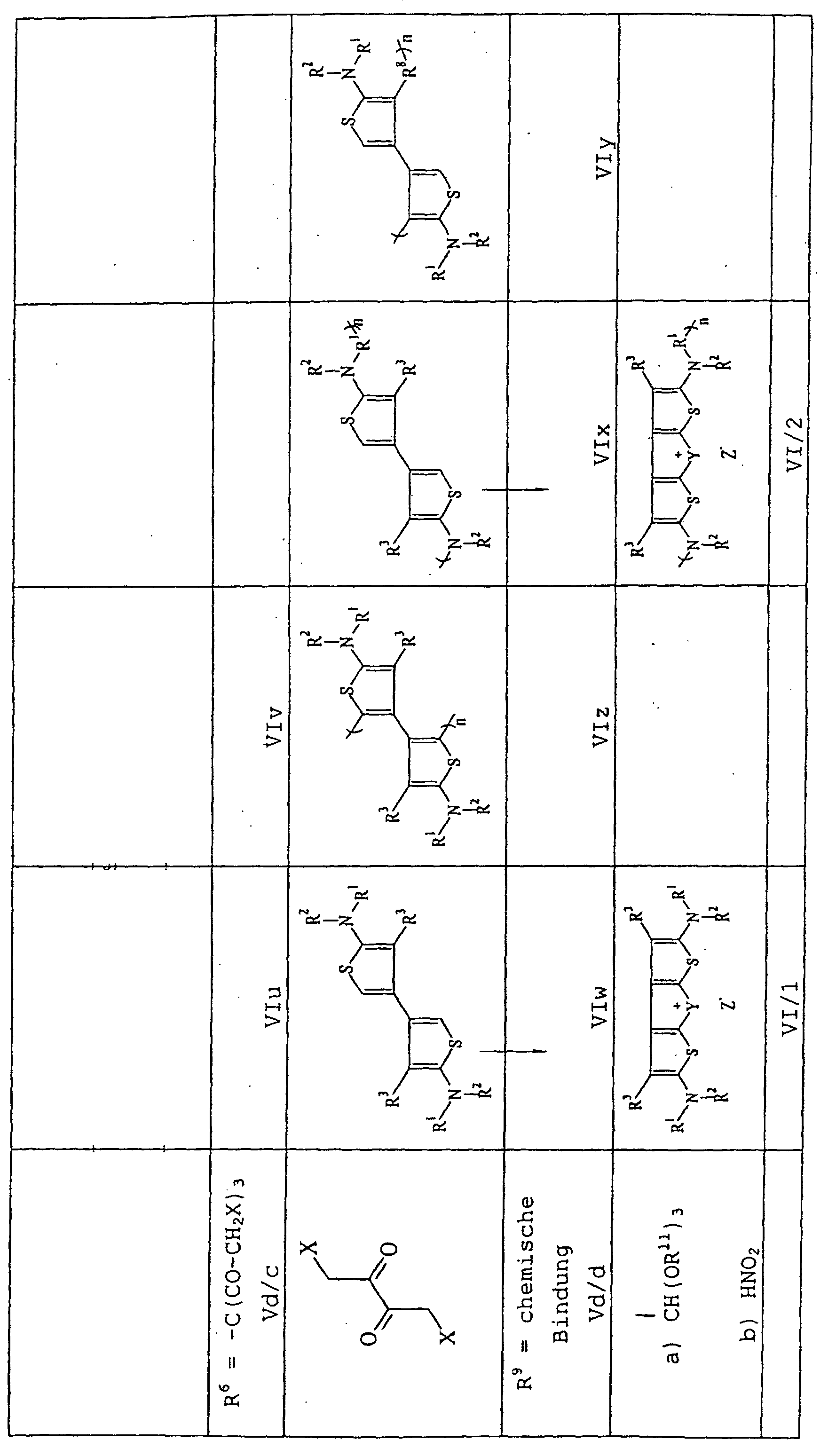

R¹¹ ist dabei ein Alkylrest mit 1 bis 5 C-Atomen, Z⁻ ist ein beliebiges Anion, vorzugsweise ein Polystyrolsulfonat oder ein anderes organisches Sulfonat, n bedeutet jeweils eine ganze Zahl von 2 bis 100.

Die Thiophen-Derivate nach der Erfindung vom Typ VI sind sämtlich geeignete Materialien für den Aufbau von organischen lichtemittierenden Dioden (OLEDs) und organischen photovoltaischen Bauelementen bzw. Zellen. Sie können sowohl in Lochtransportschichten oder Schichtkaskaden als auch in Emitter- und Elektronentransportschichten Verwendung finden. Die jeweilige Schichtposition in OLEDs wird vor allem durch die (Het)aryl- bzw. (Het)arylen-Glieder festgelegt: je mehr dieser Glieder über einen π-Elektronenmangel verfügen, d.h. so genannte π-Unterschussaromaten darstellen, desto besser eignen sich die Thiophen-Derivate als Emitter- und Elektronentransportmaterialien.

Über den beschriebenen Syntheseweg lassen sich sowohl OLED-Materialien realisieren, die als verdampfbare Verbindungen für so genannte "small molecule devices" geeignet sind, als auch durch Spin-coating verarbeitbare Polymermaterialien für so genannte "polymer devices"; die nicht-polymeren Materialien können dabei ebenfalls durch Spin-coating verarbeitet werden. Wegen der gemeinsamen Grundstruktur sämtlicher Materialien können vorteilhaft auch entsprechende Copolymere mit abgestimmten elektronischen Eigenschaften hergestellt werden. Ferner ist es möglich, die für den jeweiligen Verwendungszweck erforderlichen elektronischen Eigenschaften durch Mischungen entsprechender Materialien zu realisieren, die - aufgrund der strukturellen Ähnlichkeit - sehr gut miteinander verträglich sind. Derart abgestimmte Materialien erlauben daher einen Einschichtaufbau von OLEDs, was sehr vorteilhaft ist. Bemerkenswert sind auch die im Vergleich zu bekannten carbocyclischen Ladungstransportmaterialien sehr hohen Glasübergangstemperaturen der auf die beschriebene Weise hergestellten Ladungstransport- und Emittermaterialien, die im allgemeinen etwa 50 bis 100°C höher liegen als diejenigen der analogen carbocyclischen Verbindungen.

Anhand von Ausführungsbeispielen soll die Erfindung näher erläutert werden.

### Beispiel 1

### Synthese von Carbonsäureamiden III

In einem 2 1-Dreihalskolben mit Rückflusskühler, Magnetrührer, Tropftrichter und Inertgasdurchfluss wird jeweils 1 mol eines sekundären Amins I in 600 ml Dioxan gelöst. Das jeweils benötigte Carbonsäurehalogenid II wird danach in äquivalenter Menge zugetropft. Anschließend wird das Reaktionsgemisch unter Rückfluss erhitzt, bis die gesamte Menge des bei der Reaktion entstehenden Hydrohalogenids vom Inertgasstrom entfernt worden ist. Durch dünnschichtchromatographische Kontrolle kann das Ende der Reaktion zusätzlich detektiert werden. Die Reaktionslösung wird dann abgekühlt und in mindestens die 2fache Menge Wasser eingerührt. Dabei scheidet sich in den meisten Fällen ein Öl ab, welches nach einigen Stunden erstarrt ist. Die wässrige Phase wird abgetrennt und das Rohprodukt aus Ethanol umkristallisiert.
Die Ausbeute beträgt jeweils mindestens 90%. 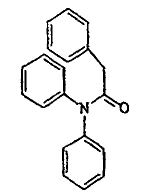 Auf diese Art wird beispielsweise 2-Phenylessigsäurediphenylamid (Fp.:71 -72°C) aus Diphenylamin und 2-Phenylacetylchlorid hergestellt.

### Beispiel 2

### Synthese von Thiocarbonsäureamiden IV

0,5 mol des jeweiligen Carbonsäureamids III und die äquivalente Menge Lawesson-Reagenz (hergestellt aus Anisol und Phosphorpentasulfid) werden in einer Rückflussapparatur mit Inertgasdurchfluss in 750 ml Diglykoldiethylether suspendiert, und dann wird 6 h bei 100°C gerührt. Dabei bildet sich eine klare Lösung, aus der in der Kälte in einigen Fällen das Reaktionsprodukt auskristallisiert. Um das Produkt vollständig zu isolieren, wird die Reaktionsmischung in die doppelte Menge Wasser eingerührt, die sich oft bildende ölige Phase lässt man dann kristallisieren. Danach wird das Produkt von der wässrigen Phase abgetrennt und aus Methanol umkristallisiert. Die Ausbeute beträgt jeweils mindestens 90%. 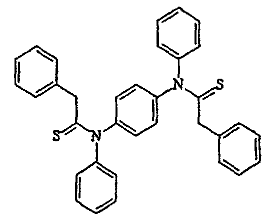 Auf diese Art wird beispielsweise das Thiocarbonsäureamid IVb (R¹ = 1,4-Phenylen und R² = R³ = Phenyl) (Fp.: 225 - 227°C, M⁺ =528) aus dem Carbonsäureamid III (R¹ = 1,4-Phenylen und R² = R³ = Phenyl) hergestellt.

### Beispiel 3

### Synthese von Thiophen-Derivaten VI

0,1 mol des jeweiligen Thiocarbonsäureamids IV werden in einem Kolben, der mit Rührer und Rückflusskühler versehen ist, zusammen mit der äquivalenten Menge einer α-Halogenacylverbindung V in 200 ml DMF gelöst, dann wird 1 h auf 100°C erwärmt. Danach werden 0,1 mol Triethylamin zugefügt und es wird weitere 30 min erwärmt. Nach dem Abkühlen wird das gebildete Thiophen-Derivat durch Fällen mit Ethanol, in manchen Fällen auch mit Wasser, und Absaugen isoliert. Das Rohprodukt wird durch Lösen in THF und Ausfällen mit Ethanol gereinigt. Die Ausbeute beträgt jeweils zwischen 60 und 80%. 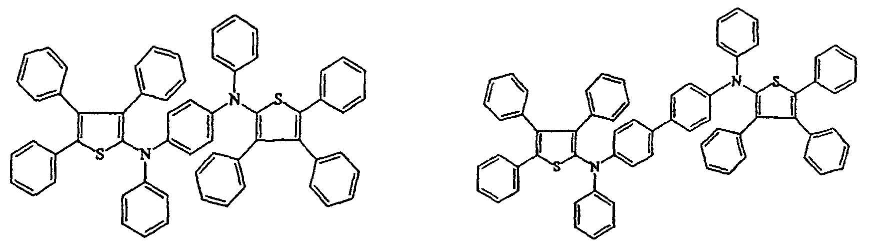 Auf diese Art wird beispielsweise das Thiophen-Derivat VIb (R¹ = 1,4-Phenylen und R² = R³ = R⁴ = R⁵ = Phenyl) (Fp.:318°C. T_{g} = 275°C, M⁺ = 880) aus dem Thiocarbonsäureamid IVb (R¹ = 1,4 Phenylen und R² = R³ = Phenyl) und Desylchlorid (α-Chlor-α-phenylacetophenon) hergestellt sowie das Thiophen-Derivat VIb (R¹ = 1,4-Biphenylen und R² = R³ = R⁴ = R⁵ = Phenyl) (Fp.:285°C °C. T_{g} = 270°C, M⁺ = 956) aus dem Thiocarbonsäureamid IVb (R¹ = 1,4 Biphenylen und R² = R³ = Phenyl) und Desylchlorid (α-Chlor-α-phenylacetophenon).

Zur Herstellung von polymeren Thiophen-Derivaten VI, beispielsweise VIh (R¹ = 1,4-Phenylen und R² = R³ = R⁴ = Phenyl), wird folgendermaßen vorgegangen: 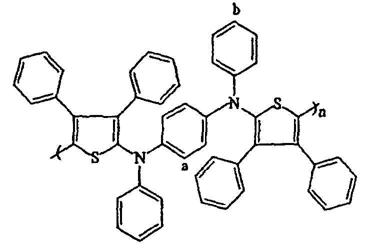 ¹H-NMR: a = 6,90ppm (d), b = 7,10ppm (t), (DMF-D₆)
0,1 mol Thiocarbonsäureamid IVb (R¹ = 1,4-Phenylen, R² = R³ = Phenyl) werden in einem Kolben, der mit Rührer und Rückflusskühler versehen ist, zusammen mit 0,1 mol dimerem Phenacylbromid Vc (R² = Phenyl) in 200 ml DMF gelöst, dann wird 1 h auf 100°C erwärmt. Danach werden - zur Verkappung der Endgruppen - nacheinander ein monofunktionelles vinyloges Thioamid und ein monofunktionelles Acylhalogenid zugegeben, im vorliegenden Fall zunächst 0,01 mol 2-Phenylthioessigsäurediphenylamid und nach 60 min 0,01 mol Phenacylbromid. Nach weiteren 60 min werden 0,1 mol Triethylamin zugesetzt und nach weiteren 15 min lässt man abkühlen, wobei das gebildete polymere Thiophen-Derivat ausfällt. Die weitere Aufarbeitung erfolgt in der vorstehend beschriebenen Weise; Ausbeute: ca. 80 %.

### Beispiel 4

### Synthese von dimeren bzw. polymeren Thiophen-Derivaten VI (durch oxidative Kupplung)

0,01 mol eines in 5-Stellung unsubstituierten Thiophen-Derivates VI werden in einem Kolben, der mit Rückflusskühler, Rührer, Feststoffdosiereinrichtung und Inertgasdurchfluß versehen ist, in 100 ml getrocknetem THF gelöst. Es wird auf -60°C abgekühlt, und dann werden 0,015 mol Butyllithium zugesetzt. Anschließend wird die Kühlung entfernt und man lässt das Reaktionsgemisch bis auf -10°C auftauen. Nachfolgend werden mittels der Feststoffdosiereinrichtung 0,011 mol Kupfer-II-chlorid zugefügt, und dann wird weiter bis auf 40°C erwärmt. Die Reaktion wird nach 30 min abgebrochen, indem das Produkt mit Wasser (bei Polymeren mit Methanol mit einem Zusatz von 10% Wasser) ausgefällt und danach abgesaugt wird. Durch mehrfaches Lösen in THF und Ausfällen mit Methanol wird das Produkt gereinigt. Nicht-polymere Verbindungen können auch durch Sublimation gereinigt werden. 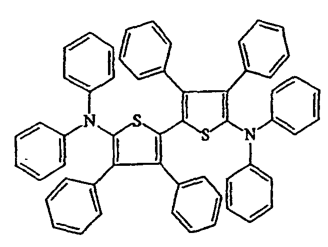 Auf diese Art wird beispielsweise das dimere Thiophen-Derivat VIg (R¹ = R² = R³ = R⁴ = Phenyl) (Fp.: 255 - 258°C, M⁺ = 804) aus 2-Diphenylamino-3,4-diphenyl-thiophen VId hergestellt.

### Beispiel 5

### Synthese von Thiophen-Derivaten VI in Form kationisch leitfähiger Materialien (Y = CH)

0,01 mol eines in 4,4'-Stellung verbundenen und in 5,5'-Stellung unsubstituierten dimeren Thiophen-Derivates VI werden in einem Becherglas in 100 ml DMF gelöst und mit 0,015 mol Orthoameisensäuretriethylester versetzt. Nach der Zugabe von 0,01 mol Perchlorsäure und Erwärmen auf ca. 100°C entsteht ein im langwelligen Bereich absorbierendes Farbsalz, welches nach der Zugabe von Ethanol und eventuell etwas Ether ausgefällt und dann abgesaugt wird. Durch Umsetzung des Farbstoff-Perchlorats mit einer Lösung von Natrium-polystyrolsulfonat wird eine wässrige Lösung des jeweiligen kationisch leitfähigen Materials als Polystyrolsulfonat erhalten; diese Lösung kann durch Spin-coating verarbeitet werden. 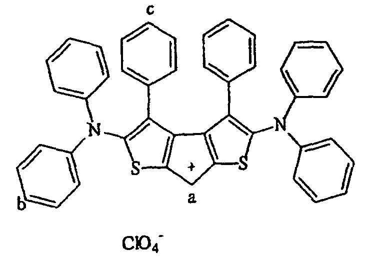 ¹H-NMR: a = 7,20ppm (s), b = 7,60ppm (t), c = 7,40ppm (t) (DMSO-D₆)
λₘₐₓ = 687nm
Auf diese Art wird beispielsweise das Thiophen-Derivat VI/1 (R¹ = R² = R³ = Phenyl und Y = CH) (M⁺ = 663) aus dem Thiophen-Derivat VIw (R¹ = R² = R³ = Phenyl) und Orthoameisensäuretriethylester in Gegenwart von Perchlorsäure hergestellt.

### Beispiel 6

### Synthese von Thiophen-Derivaten VI in Form kationisch leitfähiger Materialien (Y = N)

0,01 mol eines in 4,4'-Stellung verbundenen und in 5,5'-Stellung unsubstituierten dimeren Thiophen-Derivates VI werden in einem Becherglas in 100 ml THF gelöst und mit 0,015 mol Isoamylnitrit versetzt. Nach der Zugabe von 0,01 mol Perchlorsäure unter Kühlen und nachfolgendem Erwärmen auf ca. 50°C entsteht ein im langwelligen Bereich absorbierendes Farbsalz, welches nach der Zugabe von Ethanol und eventuell etwas Ether ausgefällt und dann abgesaugt wird. Durch Umsetzung des Farbstoff-Perchlorats mit einer Lösung von Natriumpolystyrolsulfonat wird eine wässrige Lösung des jeweiligen kationisch leitfähigen Materials als Polystyrolsulfonat erhalten; diese Lösung kann durch Spin-coating verarbeitet werden. 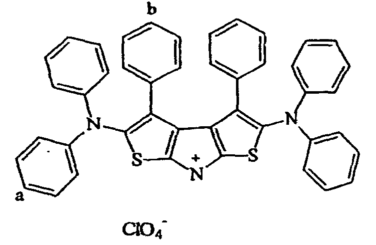 ¹H-NMR: a = 7,65ppm (t) , b = 7, 55ppm (t) , (DMSO-D₆)
λₘₐₓ = 750nm
Auf diese Art wird beispielsweise das Thiophen-Derivat VI/1 (R¹ = R² = R³ = Phenyl und Y = N) (M⁺ =664) aus dem Thiophen-Derivat VIw (R¹ = R² = R³ = Phenyl) und Isoamylnitrit in Gegenwart von Perchlorsäure hergestellt.

## Patentansprüche

1. 2-(N,N-Di(het)arylamino)-thiophen Derivate der allgemeinen Strukturen 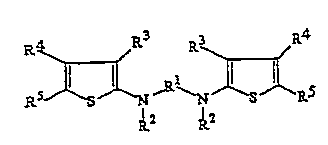 und 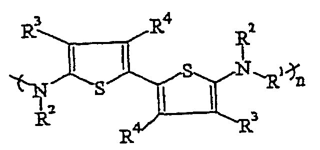 wobei Folgendes gilt:
R², R³, R⁴ und R⁵ sind - unabhängig voneinander - jeweils ein monofunktionelles (Het)arylsystem in Form eines konjugierten carbocyclischen oder heterocyclischen Ringsystems, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylamiogruppen (Alkyl = C₁ bis C₆) substituiert sein können und R¹ ist ein entsprechendes bifunktionelles (Het) arylensystem, und
wobei n = 2 bis 100 und eine ganze Zahl ist.

2. 2-(N,N-Di(het)arylamino)-thiophen Derivate folgender Strukturen:

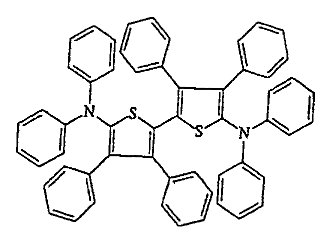

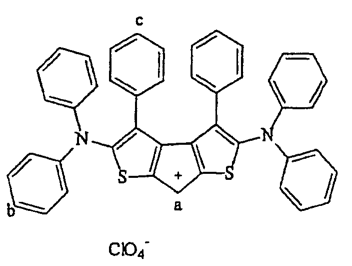

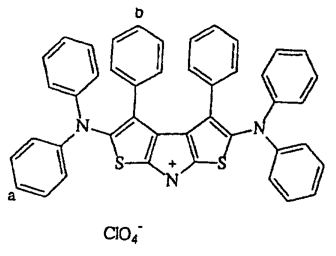

3. Verfahren zur Herstellung von Thiophen-Derivaten nach einem der Ansprüche 1 oder 2, **gekennzeichnet durch** folgende Schritte:
(a) Umsetzung eines sekundären Amins mit einem Carbonsäurehalogenid zu einem Carbonsäureamid;
(b) Überführung des Carbonsäureamids in ein Thiocarbonsäureamid;
(c) Umsetzung des Thiocarbonsäureamids mit einer α-Halogenacyl-Verbindung zu einem 2-(N,N-Di(het)arylamino)-thiophen-Derivat.

4. Verfahren nach Anspruch 3, **dadurch gekennzeichnet, dass** das Thiophen-Derivat oxidativ in ein Dimer oder Polymer überführt wird.

5. Verfahren nach Anspruch 3, **dadurch gekennzeichnet, dass** das Thiophen-Derivat mittels Orthoameisensäureester oder salpetriger Säure in ein Polythiophen-Derivat überführt wird.

6. Verwendung der Thiophen-Derivate nach den Ansprüchen 1 und/oder 2 in organischen lichtemittierenden Dioden.

7. Verwendung der Thiophen-Derivate nach den Ansprüchen 1 und/oder 2 in organischen photovoltaischen Bauelementen.

## Claims

1. 2-(N,N-Di(het)arylamino)thiophene derivatives of the general structures 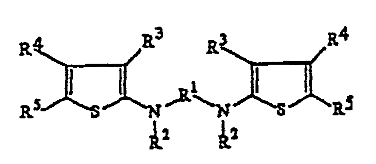 and 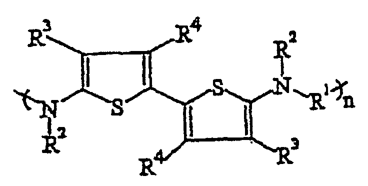 in which the following is applicable:
R², R³, R⁴ and R⁵ - independently of one another - are each a monofunctional (het)aryl system, in the form of a conjugated carbocyclic or heterocyclic ring system which may also comprise linearly or angularly annulated or linked identical or different ring types, wherein the peripheral hydrogen atoms, if necessary, can be substituted by alkyl, alkoxy, phenoxy, dialkylamino or diphenylamino groups (alkyl = C₁ to C₆) and
R¹ is a corresponding bifunctional (het)aryl system, and in which n = 2 to 100 and is an integer.

2. 2-(N,N-Di(het) arylamino) thiophene derivatives of the following structures:

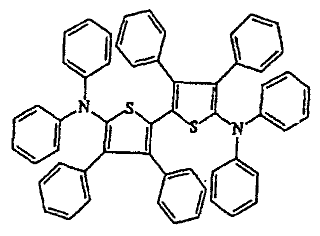

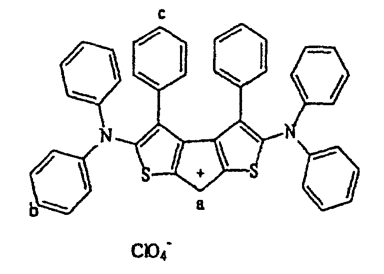

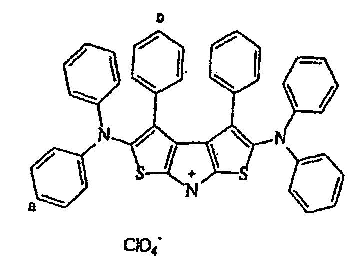

3. Process for the preparation of thiophene derivatives according to either of Claims 1 and 2, **characterized by** the following steps:
(a) reaction of a secondary amine with an acyl halide to give a carboxamide;
(b) conversion of the carboxamide into a thiocarboxamide;
(c) reaction of the thiocarboxamide with an α-haloacyl compound to give a 2-(N,N-di(het)arylamino)thiophene derivative.

4. Process according to Claim 3, **characterized in that** the thiophene derivative is converted oxidatively into a dimer or polymer.

5. Process according to Claim 3, **characterized in that** the thiophene derivative is converted by means of an orthoformic ester or nitrous acid into a polythiophene derivative.

6. Use of the thiophene derivatives according to Claims 1 and/or 2 in organic light-emitting diodes.

7. Use of thiophene derivatives according to Claims 1 and/or 2 in organic photovoltaic components.

## Revendications

1. Dérivés de 2-(N,N-di(hét)arylamino)-thiophène de structures générales 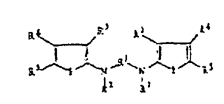 et 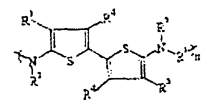 dans lesquelles on a ce qui suit :
R², R³, R⁴ et R⁵ représentent chacun - indépendamment l'un de l'autre - un système (hét)aryle monofonctionnel sous la forme d'un système cyclique carbocyclique ou hétérocyclique conjugué, qui peut également consister en types de cycles identiques ou différents, anellés ou reliés de façon linéaire ou angulaire, dans lequel les atomes d'hydrogène périphériques peuvent le cas échéant être remplacés par des groupes alkyle, alcoxy, phénoxy, dialkylamino ou diphénylamino (alkyle = C₁ à C₆) et
R¹ est un système (hét)arylène bifonctionnel correspondant, et
n = 2 à 100 et est un nombre entier.

2. Dérivés de 2-(N,N-di(hét)arylamino)-thiophène de structures suivantes

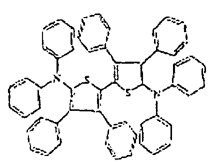

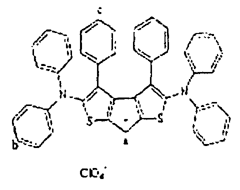

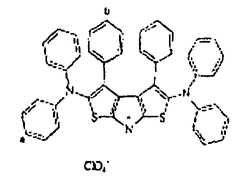

3. Procédé de préparation de dérivés de thiophène selon l'une des revendications 1 ou 2, **caractérisé par** les étapes suivantes :
(a) transformation d'une amine secondaire avec un halogénure d'acide carboxylique pour donner un amide d'acide carboxylique ;
(b) transformation de l'amide d'acide carboxylique en un amide d'acide thiocarboxylique ;
(c) transformation de l'amide d'acide thiocarboxylique avec un composé α-halogène-acyle pour donner un dérivé de 2-(N,N-di(hét)arylamino)-thiophène.

4. Procédé selon la revendication 3, **caractérisé en ce que** le dérivé thiophène est transformé par oxydation en un dimère ou en un polymère.

5. Procédé selon la revendication 3, **caractérisé en ce que** le dérivé de thiophène est transformé en un dérivé de polythiophène au moyen d'un ester de l'acide orthoformique ou d'acide nitreux.

6. Utilisation des dérivés de thiophène selon les revendications 1 et/ou 2 dans des diodes organiques émettrices de lumière.

7. Utilisation des dérivés de thiophène selon les revendications 1 et/ou 2 dans des éléments de construction photovoltaïques organiques.
